(19) Europäisches Patentamt
European Patent Office
Office européen des brevets

(11) Publication number: **0 118 036**
**A1**

(12) # EUROPEAN PATENT APPLICATION

(21) Application number: 84101070.5

(22) Date of filing: 02.02.84

(51) Int. Cl.³: C 07 C 97/10
C 07 C 57/145, A 61 K 31/135

(30) Priority: 03.02.83 GB 8302968
15.11.83 GB 8330479

(43) Date of publication of application:
12.09.84 Bulletin 84/37

(84) Designated Contracting States:
AT BE CH DE FR GB IT LI LU NL SE

(71) Applicant: THE WELLCOME FOUNDATION LIMITED
183-193 Euston Road
London NW1 2BP(GB)

(72) Inventor: Billinghurst, John Evlyn Warner
"Fairmeadow" Basted Lane
Crouch Near Sevenoaks Kent(GB)

(72) Inventor: Scharver, Jeffrey Douglas
5202, Autumn Drive
Durham North Carolina 27712(US)

(72) Inventor: Yeowell, David Arthur
608 Concordia Court
Chapel Hill North Carolina 27514(US)

(74) Representative: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

(54) **Biologically active ketone derivative, preparation and use.**

(57) 2-*tert*-Butylamino-3'-chloropropiophenone maleate, its preparation, pharmaceutical compositions containing it and their preparation, and its use in therapy, for example the treatment of depression in man.

EP 0 118 036 A1

Croydon Printing Company Ltd.

BIOLOGICALLY ACTIVE KETONE DERIVATIVE, PREPARATION AND USE

The present invention relates to a novel salt of 2-*tert*-butylamino-3'-chloropropiophenone.

2-*tert*-Butylamino-3'-chloropropiophenone and the corresponding 3'-fluoro analogue are described in our British Patent Specification No. 1,340,032 which also broadly discloses the pharmaceutically acceptable salts of these bases, the salts of the following acids being specifically mentioned in the Specification, namely hydrochloric, sulphuric, phosphoric and toluenesulphonic acids. As indicated in the Specification, the above described compounds possess valuable anti-depressant properties when tested by standard techniques used in the art for determining anti-depressant activity, for example the tetrabenazine induced sedation test in rodents. It has been found that the compounds require much larger doses for stimulant action than for anti-depressant action. The compounds are also not inhibitors of monoamine oxidase nor do they have a pressor effect. Of the two free bases and their salts mentioned above, the chloro analogue and its pharmaceutically acceptable salts have been found to have particularly advantageous antidepressant activity.

As mentioned in the Specification, 2-*tert*-butylamino-3'-chloropropiophenone and its 3'-fluoro analogue are moderately weak bases ($pK_a$ about 8.5-9) and are desirably stored and administered as pharmaceutically acceptable salts, mineral acid salts and particularly the hydrochloride salt being mentioned for this purpose in the Specification.

However, it has now been found that problems arise in the selection of suitable salts for use as therapeutic agents because of poor stability when prepared and stored in bulk and/or when used to make pharmaceutical formulations. Thus, for example, from experiments which we have carried out, we have found that the following salts of 2-tert-butylamino-3'-chloropropiophenone are unstable upon storage, namely the salts of formic, fumaric, citric, phthalic and trifluoromethanesulphonic acids, as well as the phosphate monohydrate, hemisulphate sesquihydrate, methanesulphonate sesquihydrate, p-toluenesulphonate monohydrate and napthalenesulphonate monohydrate salts. The propiophenone moiety breaks down into hydroxyketones, a diketone and meta-chlorobenzoic acid. It is of course an important desideratum for a pharmaceutical product that it should have a reasonably good bulk stability in order to avoid or reduce wastage resulting from decomposition of the active moiety during the period between its synthesis and its use in formulating the final dosage form.

A further problem is that the above-mentioned salts of the base and also the hydrochloride have limited stability when formulated with conventional pharmaceutial carriers or excipients into solid compositions. It will be appreciated that, in order for the salts to be used in medical therapy, it is necessary to formulate them into pharmaceutical compositions suitable for administration to the patient. The formulation of such compositions entails the admixture or contacting of the salts with various pharmaceutical carriers or excipients. Examples of solid pharmaceutical compositions which are mentioned in British Specification 1,340,032 are tablets, cachets, capsules and suppositories.

Of these various types of composition, tablets and capsules are particularly preferred forms as it is generally desired to administer the active compound by the oral route. However, from experiments which we have carried out, we have found that the hydrochloride salt of 2-tert-butylamino-3'-chloropropiophenone has limited stability in the presence of conventional tableting excipients, particularly magnesium stearate and stearic acid which are commonly used as lubricants in tablet formulations. The formulation of the salts of the free base in capsules also presents problems because gelatin, which is commonly used to make the capsule shell, is hydrophilic and therefore creates a humid environment within the capsule, which has a deleterious effect on the 2-tert-butylamino-3'chloropropiophenone salt present in the capsule. It will therefore be appreciated that considerable difficulty arose in finding a salt of 2-tert-butylamino-3'-chloropropiophenone which has sufficient stability when formulated in pharmaceutical compositions.

We have now discovered that the maleate salt of 2-tert-butylamino-3'-chloropropiophenone has advantageous stability characteristics in association with conventional pharmaceutical carriers and excipients, particularly carriers or excipients which are commonly employed in the formulation of solid compositions such as tablets and capsules. A further advantage of the maleate salt is that it is much less corrosive than, for example, the hydrochloride salt which tends to corrode apparatus used in its preparation and formulation. A particular problem with the hydrochloride is that it tends to corrode tablet press die tables causing considerable economic disadvantage.

According to one feature of the present invention therefore we provide 2-_tert_-butylamino-3'-chloropropiophenone maleate.

The use of the maleate salt of 2-_tert_-butylamino-3'-chloropropiophenone enables one to present the base in a substantially stable form which can be relatively easily prepared, then stored in bulk for reasonable periods of time and/or which can be formulated in pharmaceutical compositions particularly in solid compositions such as tablets and capsules, thereby avoiding the stability problems which, as discussed above, are associated with other salts. The maleate salt has been found to have good antidepressant activity when tested in the tetrabenazine-induced sedation test in rodents.

According to a further feature of the present invention we provide 2-_tert_-butylamino-3'-chloropropiophenone maleate for use in the therapeutic treatment of a mammal, for example, for use in the treatment of depression in mammals, including man.

The present invention also provides a method of treating depression in a mammal, including a human being, which comprises administering to said mammal an effective dose of 2-_tert_-butylamino-3'-chloropropiophenone maleate, for example in a form of a pharmaceutical composition as hereinafter described.

The present invention further provides a method of treating one or more of the following conditions in a mammal, including a human being, which comprises administering to said mammal a dose effective

to treat said condition of 2-tert-butylamino-3'-chloropropiophenone maleate, for example, in a form of a pharmaceutical composition as hereinafter described: 1) minimal brain dysfunction; 2) tardive dyskinesia; 3) mania; 4) hypercholesterolemia; 5) hyperprolactinemia and other conditions wherein reduced prolactin secretion is desirable, such as prolactin sensitive mammary cancer, galactorrhea, etc.; 6) mental functional impairment caused by alcohol consumption; and 7) functional impairment and drowsiness caused by the use of benzodiazepines.

According to a further feature of the present invention we provide pharmaceutical compositions comprising, as active ingredient, 2-tert-butylamino-3'-chloropropiophenone maleate in association with at least one pharmaceutical carrier or excipient. Conveniently the said active ingredient comprises 5 to 95% by weight of the composition.

The above-described compositions according to the present invention are preferably presented in solid unit dosage forms for administration by the oral or rectal route, e.g. in the form of tablets, cachets, capsules or suppositories. They may also be presented in solution or suspension for oral administration or in solution for parenteral administration.

It is particularly preferred to present the maleate salt according to the present invention in an oral dosage unit preparation (e.g. as a cachet, tablet or capsule) containing one or more pharmaceutically acceptable carriers which may take the form of solid fillers

or diluents such as lactose, starch such as corn, potato or rice starch, microcrystalline cellulose, as well as other excipients conventionally known in the pharmaceutical art for this purpose including binders, thickeners, lubricants, disintegrants, surfactants, buffers, sweetening and other flavouring agents, coloring agents, coatings and preservatives. As indicated above, a particularly preferred type of pharmaceutical composition for the administration of the maleate salt is the tablet comprising, in addition to the active ingredient, one or more of the above-mentioned pharmaceutical carriers or excipients suitable for tablet formulations, for example, inert diluents such as lactose and corn starch, and/or lubricants such as talc, hydrogenated vegetable oil, stearic acid, lubritab and magnesium stearate. Another preferred type of pharmaceutical composition is the capsule wherein the active ingredient optionally in admixture with one or more appropriate pharmaceutical carriers or excipients, e.g., selected from those described above, is presented in a capsule shell, for example, of gelatin. The gelatin capsule is preferably opaque and may be colored, generally being hard rather than soft gelatin.

The formulation of the active ingredient as suppositories for rectal administration may be effected using conventional pharmaceutically acceptable carriers for this purpose such as cocoa butter.

For the oral or rectal route of administration, the preferred dosage of the active ingredient (calculated as the base) is about 0.25 mg/kg to 5 mg/kg of mammal body weight while the most preferred dosage (calculated as the base) is about 0.75 mg/kg to 3.5 mg/kg of

mammal body weight. The active ingredient is preferably administered 3 times daily although the number of daily administrations of the medication may vary according to the subject (mammal) being treated, the nature of the formulation and the exercise of the physician's discretion.

For the treatment of humans, the preferred unit dosage of the active ingredient (calculated as the base) for oral administration, or administration as a suppository, is about 15 to 500 mg with the more preferred unit dosage being about 35 to 250 mg, and the most preferred unit dosage being about 40 to 150 mg.

The compositions of the present invention may be prepared by any of the methods of pharmacy but all methods include the step of bringing into association the active ingredient with the carrier or excipient. In general the compositions are prepared by uniformly and intimately admixing the ingredients. This may be achieved by simple admixture but preferably involves granulation, dry or wet, in the latter instance with readily available liquids such as water or alcohol. The granules with or without compaction may then be compressed or molded into tablets or suppositories, or filled into capsules or cachets. Suppositories may also be made by melting the required base, e.g. the cocoa butter, mixing in the active ingredient, and molding the mixture or allowing it to solidify, powdering or granulating this and then compressing the powder or granules into the suppository.

Other formulations suitable for oral administration include powders and granules; a bolus, electuary or paste; a solution or a suspension in an aqueous liquid or a non-aqueous liquid; and an oil-in-water or water-in-oil liquid emulsion.

The active ingredient may also be administered by the parenteral (including subcutaneous, intradermal, intramuscular and intravenous) route.

Formulations for parenteral administration include aqueous and non-aqueous sterile injection solutions which may contain anti-oxidents, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

When administered by the parenteral route the required dosage of the active ingredient (calculated as the base) will generally be one-half of that indicated <u>supra</u> as appropriate for oral administration.

Preferred unit dosage preparations are those containing a unit dosage, as hereinabove recited, or an appropriate fraction thereof, of the active ingredient.

The compositions of the present invention as above described may optionally contain other therepeutic agents to complement and/or supplement the activity of the maleate salt.

2-*tert*-Butylamino-3'-chloropropiophenone maleate may be prepared in conventional manner, for example by analogous procedures to those described in British Patent Specification, 1,340,032. In principle, the two preferred methods of preparing the maleate are:--

(a) metathesis in solution or suspension of another salt of the base with maleic acid or a functional equivalent thereof; or

(b) treatment of the free base in solution or suspension with maleic acid or a functional equivalent thereof.

With regard to process a), this may be conveniently effected by reacting the other salt of the base with maleic acid in a solvent medium, preferably in an aqueous medium. The reaction is preferably effected at a temperature of about 25-30°C.

The initial salt of the base is preferably the hydrochloride in view of its ease of preparation as described in British Patent Specification 1,340,032. The maleic acid employed in this process is preferably obtained by hydrolysis of maleic anhydride in order to avoid

contamination with the geometrically isomeric fumaric acid. Hydrochloric acid liberated in this process may be neutralised by the addition to the reaction mixture of an appropriate amount of a base, e.g. an alkali metal hydroxide such as sodium hydroxide, for example to adjust the pH of the reaction mixture to about 2.

With regard to process b), this may be conveniently effected by reacting the base with maleic acid in a solvent medium, e.g., in an aqueous or more preferably an organic solvent medium, for example, comprising one or more organic solvents selected from aromatic hydrocarbons such as toluene and alkanols such as ethanol. As in process a) above, it is preferred to obtain maleic acid by hydrolysis of maleic anhydride.

In both of processes a) and b) the maleic acid is advantageously employed in an approximately equimolar amount with respect to the base.

The base or salt thereof employed as starting material in processes a) and b) may be prepared as described in British Patent Specification 1,340,032 and, if desired, formation of the maleate salt in accordance with the present invention may be effected without isolation and/or purification of the intermediate base or salt.

A particularly preferred method described in the said British Patent Specification for the preparation of the base or a salt thereof comprises the reaction of a compound of formula (I):

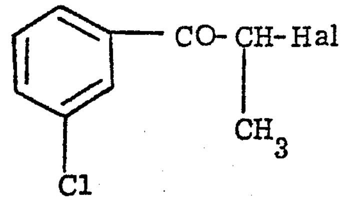

(I)

(wherein Hal is chloro, bromo or iodo) with tert-butylamine and, if desired, converting the resulting product to an acid addition salt thereof. The above-mentioned reaction is slow in the absence of solvent since tert-butylamine normally reacts very slowly with 2-halopropiophenones. It is desirable to include an organic solvent in the reaction mixture, and for this purpose acetonitrile offers marked advantages. It improves the kinetics of the reaction, is unreactive under the conditions used and is relatively low boiling. Other polar solvents, protic or aprotic, may be used, for example lower aliphatic ketones (i.e. those having from 1 to 4 carbon atoms in each alkyl group) or ethers, but the reaction is slow in these solvents. Others which may be used include dimethylformamide, dimethylacetamide, nitromethane, dimethylsulphoxide and hexamethylphosphoramide.

It may be desirable to heat the reactants, for example at the reflux temperature of the reaction mixture. The amine is preferably present in excess relative to the compound of formula (I); up to five times the equimolar quantity may be used. If Hal in formula (I) is chloro, then a catalytic amount of an iodide salt, for example sodium iodide may be included with advantage in the reaction mixture.

The following Examples illustrate the present invention, but should not be taken to in any way constitute a limitation thereof.

EXAMPLE 1

2-*tert*-Butylamino-3'-chloropropiophenone hydrochloride (83 g, 0.3 mole) was dissolved in 0.5 L of water and an equimolar quantity of aqueous sodium hydroxide was added. The free base of the α-amino-ketone was extracted by washing with toluene (200 mL). The organic extract was diluted with an equal volume of toluene and treated dropwise with a solution of maleic acid (35 g, 0.3 mole) in ethanol (150 mL). After 30 minutes the precipitated maleate salt was removed by filtration, washed with cold toluene, and dried *in vacuo* at 50°C to give 2-*tert*-butylamino-3'-chloropropiophenone maleate as a white solid: 99.7 g (93.2%), m.p.. 180-183°C; NMR(60 MHz, DMSO-$d_6$) δ 8.3-7.6 (m, 4H, aromatic), 6.0 (s, 2H, vinyl), 5.3 (q, 1H, methine), 1.5 (d, 3H, methyl), 1.3 (s, 9H, *tert*-butyl).

Anal. calcd. for $C_{17}H_{22}NO_5Cl$: C, 57.38; H, 6.23; N, 3.94; Cl 9.94.
Found: C, 57.47; H, 6.25: N, 3.91; Cl, 9.87.

EXAMPLE 2

2-Bromo-3'-chloropropiophenone (48 g, 0.19 mole) in acetonitrile (60 mL) was treated dropwise with *tert*-butylamine (35 g, 0.48 mole) over one hour keeping the temperature below 30°C. After an additional one hour the mixture was stripped *in vacuo* and taken up in toluene (100 mL). The insoluble *tert*-butylamine hydrobromide was removed by filtration and washed with toluene. The combined organic phases were extracted twice with excess cold aqueous hydrochloric acid and the aqueous extracts were treated with Darco G-60 for 2 hours at room

temperature; then filtered and diluted to 400 mL with water. This solution was treated with an aqueous solution of maleic acid (22.6 g, 0.19 mole) with good stirring. The mixture was neutralized to pH 2 by the dropwise addition of 40% aqueous sodium hydroxide and the precipitated maleate was removed by filtration and washed with water. After slurrying with water, to remove residual ionic chloride, the cake was dried *in vacuo* at 50°C to give 2-*tert*-butylamino-3'-chloropropiophenone maleate as a white powder: 48.6 g (70.4%), m.p. 179-181°C; NMR(100 MHz, DMSO-$d_6$) $\delta$ 8.35-7.60 (m, 4H, aromatic), 6.05 (s, 2H, vinyl), 5.30 (q, 1H, methine), 1.45 (d, 3H, methyl), 1.30 (s, 9H, *tert*-butyl).

Anal. for $C_{17}H_{22}NO_5Cl$: Calculated: C, 57.38; H, 6.23; N, 3.94; Cl, 9.94; Found: C, 57.45; H, 6.26; N, 3.92; Cl, 9.94.

EXAMPLE 3

Tablets were prepared having the following composition:-

| Ingredient | mg/tablet |
|---|---|
| Active ingredient | 74.21* |
| Lactose | 180.79 |
| Starch, corn | 30.00 |
| Methylcellulose (400 cps) | 3.00 |
| Magnesium stearate | 3.00 |
| Total weight | 291.00 |

* equivalent to 50 mg of base.

A wet granulation formulation using the methylcellulose as binder was employed to make tablets of 291 mg theoretical compression weight on a single punch tablet press. The resulting tablets were 9.4 mm round, biconvex.

EXAMPLE 4

Tablets were prepared as described in Example 3 except that 12 mg of talc was substituted for the magnesium stearate, the resulting theoretical compression weight of the tablets being 300 mg.

EXAMPLE 5

Tablets were prepared having the following compositions:-

| Ingredient | mg/tablet A | mg/tablet B |
|---|---|---|
| Active ingredient | 65* | 195** |
| Microcrystalline cellulose | 281 | 151 |
| Starch, corn | 30 | 30 |

| | | |
|---|---|---|
| Methylcellulose | 8 | 8 |
| Talc | 8 | 8 |
| Stearic acid | 8 | 8 |
| Total weight | 400 | 400 |

* equivalent to 44 mg of base

** equivalent to 131 mg of base

Method

The active ingredient, diluent and starch were mixed together and then granulated with an aqueous solution of methyl cellulose. After drying the granules, the talc and stearic acid were blended in and tablets compressed at an average weight of 400 mg.

EXAMPLE 6

Capsules were prepared having the following composition:-

| Ingredient | mg/capsule |
|---|---|
| Active ingredient | 97.5* |
| Lactose | 111.5 |
| Methylcellulose 400 cps | 3.0 |
| Starch | 24.0 |
| Talc | 4.0 |
| Total fill weight | 240.0 |

* equivalent to 66 mg of base

The active ingredient, lactose and starch were mixed together and then granulated with a solution of the methylcellulose in water. After drying the granules, the talc was blended in and filled into hard, opaque, gelatin capsules to an average fill weight of 240 mg.

EXAMPLE 7

Capsules were prepared having the following composition:-

| Ingredient | mg/capsule |
|---|---|
| Active ingredient | 130* |
| Spray Dried Lactose | 99 |
| Sodium Starch Glycolate | 7 |
| Talc | 4 |
| Total fill weight | 240 |

* equivalent to 88 mg of base

The active ingredient, spray dried lactose, sodium starch glycolate and talc were blended together and filled into hard opaque gelatin capsules to an average fill weight of 240 mg.

EXAMPLE 8

Hard, opaque, gelatin capsules were filled with the indicated ingredients (mg/capsule):

| Ingredient | Formulae | | | |
|---|---|---|---|---|
| | (A) | (B) | (C) | (D) |
| Active ingredient | 64.41 | 96.61 | 128.80 | 193.20 |
| (equivalent to base) | (43.40) | (65.09) | (86.78) | (130.17) |
| Corn starch NF | 48.00 | 39.00 | 52.00 | 62.00 |
| Lactose, hydrous USP | 343.59 | 194.39 | 259.20 | 325.80 |

| | | | | |
|---|---|---|---|---|
| Talc USP | 24.00 | 21.00 | 28.00 | 34.00 |
| Total fill weight | 480.00 | 351.00 | 468.00 | 615.00 |
| Capsule size | 1 | 1 | 1 | 0 |

EXAMPLE 9 Comparative stability of various salts of 2-tert-butylamino-3'-chloropropiophenone

Small amounts of various salts of 2-*tert*-butylamino-3'-chloropropiophenone were each placed in a clean borosilicate glass vial which was sealed with a Teflon-faced rubber closure. The samples were then placed in an oven maintained at 70°C (except for the formate which was stored at 75°C) and visual comparisons were made over a period of 1-3 days. The observations are recorded in the following Table I:

TABLE I

| Salt | Observation |
|---|---|
| Hydrochloride | No visible decomposition was observed over a period of 3 days. |
| Maleate | No visible decomposition was observed over a period of 3 days. |
| Fumarate | Extensive decomposition after 3 days as shown by severe discoloration and some fusion. |
| Citrate | Extensive decomposition after 3 days as shown by severe discoloration and some fusion. |
| Methanesulphonate sesquihydrate | Extensive decomposition was observed over a period of several hours. |
| Trifluoromethanesulphonate | Extensive decomposition after 3 days as shown by severe discoloration and some fusion. |
| Phosphate mononydrate | Extensive decomposition after 3 days as shown by severe discoloration and some fusion. |

| | |
|---|---|
| Formate | Extensive decomposition after 1 day as shown by severe discoloration. |

EXAMPLE 10

Comparative stability of tablets of Examples 3 and 4

In order to compare the storage stability of the tablets of Examples 3 and 4 with similar tablets containing the hydrochloride salt of 2-*tert*-butylamino-3'-chloropropiophenone, tablets having the following compositions were prepared in an analogous manner to that described in Examples 3 and 4:-

| Ingredient | mg/tablet | |
|---|---|---|
| | 10A | 10B |
| 2-*tert*-Butylamino-3'-chloropropiophenone hydrochloride | 50.0 | 50.0 |
| Lactose | 205.00 | 205.00 |
| Corn starch | 30.00 | 30.00 |
| Methylcellulose (400 cps) | 3.00 | 3.00 |
| Magnesium stearate | 3.00 | ---- |
| Talc | ----- | 12.00 |
| Total weight | 291.00 | 300.00 |

The four sets of tablets (3, 4, 10A and 10B) were tested for stability. Testing was carried out on tablets:

(a) stored at 50°C in closed bottles; and

(b) stored at 37°C in open bottles under a relative humidity of 75%.

The results are summarised in the following Table II in which the data are assay values expressed as percentage of labeled strength of the active salt, and "mo." indicates month:

TABLE II

| Example | 3 | 4 | 10A | 10B |
|---|---|---|---|---|
| 1 mo./37°C. 75% RH | 97.1 | 99.3 | * | 83.3 |
| 1 mo./50°C. | 97.4 | 100.1 | 18 | 66.4 |
| 3 mo./50°C. | 90.0 | 100.5 | * | * |

*Samples not assayed because physical appearance of tablets showed gross signs of extreme chemical degradation.

The data given in Table II indicate that the hydrochloride salt had limited stability in the formulated tablets (Examples 10A and 10B), the instability being especially pronounced in the tablet containing magnesium stearate as lubricant. In contrast, the maleate salt was relatively stable, only comparatively slight decomposition being noted in the case when magnesium stearate was employed as lubricant.

EXAMPLE 11

| Ingredient | mg/tablet |
|---|---|
| Active ingredient | 74.21* |
| Corn Starch | 37.00 |
| Ethylcellulose, 10 cps | 12.00 |
| Lactose, hydrous | 500.00 |
| Talc | 15.00 |
| Alcohol, SD3A, anhydrous | q.s. |
| Total weight | 638.21 mg |

*Equivalent to 50 mg of free base.

The active ingredient, starch and lactose were mixed together, then granulated with an alcoholic solution of ethylcellulose. After drying the granules, the talc was blended in and tablets compressed at an average weight of 638.21 mg.

EXAMPLE 12

| Ingredient | mg/tablet |
|---|---|
| Active ingredient | 74.21* |
| Microcrystalline cellulose | 500.00 |
| Talc | 12.00 |
| Total weight | 586.21 mg |

*Equivalent to 50 mg of free base.

The active ingredient, microcrystalline cellulose and talc were mixed

together. Tablets were compressed at an average weight of 586.21 mg.

EXAMPLE 13

| Ingredient | mg/tablet |
| --- | --- |
| Active ingredient | 193.2* |
| Pregelatinized Starch | 387.8 |
| Hydrogenated vegetable oil | 12.0 |
| Total weight | 593.0 mg |

*Equivalent to 130 mg free base.

The active ingredient, pregelatinized starch and hydrogenated vegetable oil were mixed together. Tablets were compressed at an average weight of 593 mg.

EXAMPLE 14

| Ingredient | mg/tablet |
| --- | --- |
| Active ingredient | 128.8* |
| Lactose, hydrous | 259.2 |
| Corn Starch | 52.0 |
| Talc | 28.0 |
| Total fill weight | 468.0 mg |

*Equivalent to 86.8 mg free base.

The active ingredient, lactose, corn starch and talc were mixed together and filled into opaque hard shell gelatin capsules to an average fill weight of 468 mg.

EXAMPLE 15

Capsules were prepared as described in Example 14 except that 10 mg stearic acid was substituted for the talc. The resulting theoretical capsule fill weight being 450 mg.

EXAMPLE 16

Capsules were prepared as described in Example 14 except that 9 mg hydrogenated vegetable oil was substituted for the talc. The resulting theoretical capsule fill weight being 449 mg.

EXAMPLE 17

| Ingredient | mg/tablet |
|---|---|
| Active ingredient | 193.2* |
| Pregelatinized Starch | 387.8 |
| Talc | 34.0 |
| Total fill weight | 615.0 mg |

*Equivalent to 130 mg free base.

The active ingredient, pregelatinized starch and talc were mixed together and filled into opaque hard shell gelatin capsules to an average fill weight of 615 mg.

EXAMPLE 18

| Ingredient | mg/tablet |
|---|---|
| Active ingredient | 193.2* |
| Microcrystalline cellulose | 313.8 |
| Corn Starch | 60.0 |
| Talc | 33.0 |
| Total fill weight | 600.0 mg |

*Equivalent to 130 mg free base.

The active ingredient, microcrystalline cellulose, corn starch, and talc were mixed together and filled into opaque hard shell gelatin capsules to an average fill weight of 600 mg.

EXAMPLE 19

To improve granule flow properties, a compactor may be used to dry granulate formulations cited in Examples 3 through 8.

EXAMPLE 20

The following capsule formulations were prepared to compare the storage stability of capsules of the maleate and hydrochloride salts of 2-tert-butylamino-3'-chloropropiophenone.

| Ingredient | A | B | C |
|---|---|---|---|
| 2-tert-butylamino-3'-chloropropiophenone maleate | 130 | - | - |
| 2-tert-butylamino-3'-chloropropiophenone hydrochloride | - | 130 | 100.9* |
| Lactose | 260 | 260 | 260 |
| Corn starch | 52 | 52 | 52 |
| Talc | 26 | 26 | 26 |
| Total fill weight | 468 | 468 | 438.9 |

*Molar equivalent of 130 mg of the maleate salt

The appropriate salt was sifted through a 30 mesh screen and blended with the lactose and corn starch each of which had been sifted previously through a 60 mesh screen. Half of the talc was sifted through a 30 mesh screen and blended with the blended powders. The final blends were compacted and milled to pass through a 30 mesh screen. The remaining talc was sifted (30 mesh) over the milled powders, blended and filled into No. 1, opaque white, hard gelatin capsules.

Stability testing was carried out on the capsules:

(a) stored at 40°C and 50°C in closed polyethylene bottles; and

(b) stored at 40°C in open polyethylene bottles under a relative humidity of 75%.

The results are summarized in Table III in which the data are assay values expressed as percentage of labeled strength of the active salt.

TABLE III

| Storage Conditions | 20 A | 20 B | 20 C |
|---|---|---|---|
| Initial | 98.6 | 99.7 | 97.9 |
| 3 weeks at 50°C | 99.4 | 60.5 | 71.4 |
| 6 weeks at 50°C | 99.2 | 49.2 | 66.1 |
| 3 months at 40°C | 98.2 | 84.6 | 82.3 |
| 3 months at 50°C | 96.8 | 41.1 | 64.4 |
| 6 weeks at 40°C/75% RH | 97.9 | 19.3 | 13.2 |
| 3 months at 40°C/75% RH | 95.0 | ** | ** |

**Unfit for assay

EXAMPLE 21

Antitetrabenazine Activity

Antagonism of tetrabenazine induced sedation was measured using a modification of the method of Vernier, *et al.*, First Hahnemann Symposium

on Psychosomatic Medicine, ed. Nodim and Moyer, pub. Lea and Febiger, Philadelphia, 1962.

Mice, three groups of 12 CD1 males each, were injected intraperitoneally (ip) with a solution of 2-*tert*-butylamino-3'-chloropropiophenone hydrochloride (Cpd A) or 2-*tert*-butylamino-3'-chloropropiophenone maleate (Cpd B) or with saline. Thirty minutes later each of the mice was injected ip with a solution containing 35 mg/kg tetrabenazine hydrochloride. Thirty minutes after the injection of tetrabenazine each mouse was examined for its level of exploratory behavior which was scored on a modification of the arbitrary scale defined by Vernier, *et al*. The dose of tetrabenazine used was sufficient to cause 90 to 100 percent of the mice to remain motionless even when placed in a new environment. In the test results reported 100 percent would represent total reversal of tetrabenazine effects.

Test Results

| Compound | Dose, mg/kg (ip) | Percent Antagonism of Tetrabenazine Induced Sedation |
|---|---|---|
| Cpd A | 12.5 | 17.5 |
| | 25 | 50 |
| | 50 | 62.5 |
| Cpd B | 16.1 | 27.5 |
| | 32.2 | 62.5 |
| | 64.4 | 60 |
| Saline | 0 | 10 |

The doses of Cpd B are equivalent to the corresponding doses of Cpd A on a molar basis.

EXAMPLE 22

Toxicity Data

2-*Tert*-butylamino-3'-chloropropiophenone maleate was administered by gavage in 0.5 percent carboxymethylcellulose to Charles River CD1 mice (four males and four females) at a dose of 260 mg/kg per day for 14 days. All of the mice survived this regimen.

In EXAMPLES 3-8 and 11-19 above "Active ingredient" is 2-*tert*-butylamino-3'-chloropropiophenone maleate.

What we claim is:

1. 2-tert-Butylamino-3'-chloropropiophenone maleate.

2. 2-tert-Butylamino-3'-chloropropiophenone maleate (1:1).

3. A compound according to either of claims 1 and 2, for use in human or veterinary medicine.

4. A compound according to either of claims 1 and 2, for use in the treatment of depression in man.

5. A compound according to either of claims 1 and 2, for use in the treatment in man of a condition selected from:

- minimal brain dysfunction
- tardive dyskinesia
- mania
- hypercholesterolaemia
- hyperprolactinaemia and other conditions wherein reduced prolactin secretion is desirable
- mental functional impairment caused by ethanol consumption, and
- functional impairment and drowsiness caused by the use of benzodiazepines.

6. A pharmaceutical composition comprising a compound according to either of claims 1 and 2 together with an acceptable carrier therefor.

7. A composition according to claim 6 in unit dosage form.

8. A method for the preparation of a composition according to either of claims 6 and 7 comprising admixture of the ingredients thereof.

9. A method for the preparation of 2-tert-butylamino-3'-chloropropiophenone maleate according to either of claims 1 and 2, comprising reacting 2-tert-butylamino-3'-chloropropiophenone or another salt thereof with maleic acid or a functional equivalent thereof.

10. 2-tert-Butylamino-3'-chloropropiophenone maleate according to either of claims 1 and 2, when prepared by the method of claim 9.

11. Use of 2-tert-butylamino-3'-chloropropiophenone maleate according to either of claims 1 and 2 in human or veterinary medicine.

12. Use of 2-tert-butylamino-3'-chloropropiophenone maleate according to either of claims 1 and 2 for the treatment of depression in man.

13. Use of 2-tert-butylamino-3'-chloropropiophenone maleate according to either of claims 1 and 2 for the treatment in man of a condition selected from:

- minimal brain dysfunction
- tardive dyskinesia
- mania
- hypercholesterolaemia
- hyperprolactinaemia and other conditions wherein reduced prolactin secretion is desirable

- mental functional impairment caused by ethanol consumption, and

- functional impairment and drowsiness caused by the use of benzodiazepines.

14. Use of 2-tert-butylamino-3'-chloropropiophenone maleate according to either of claims 1 and 2 for the preparation of compositions effective in human or veterinary medicine.

15. Use of 2-tert-butylamino-3'-chloropropiophenone maleate according to either of claims 1 and 2 for the preparation of compositions effective for the treatment of depression in man.

16. Use of 2-tert-butylamino-3'-chloropropiophenone maleate according to either of claims 1 and 2 for the preparation of compositions effective for the treatment in man of a condition selected from:

- minimal brain dysfunction
- tardive dyskinesia
- mania
- hypercholesterolaemia
- hyperprolactinaemia and other conditions wherein reduced prolactin secretion is desirable
- mental functional impairment caused by ethanol consumption, and
- functional impairment and drowsiness caused by the use of benzodiazepines.

European Patent Office

# EUROPEAN SEARCH REPORT

0118036

Application number

EP 84 10 1070

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
| --- | --- | --- | --- |
| Y | US-A-3 622 675 (H. KOPPE et al.) * Claims; column 4, lines 16-26; example 38 * --- | 1-16 | C 07 C 97/10<br>C 07 C 57/145<br>A 61 K 31/135 |
| D,Y | GB-A-1 340 032 (WELLCOME) * Claims * ----- | 1-16 | |
| | | | TECHNICAL FIELDS SEARCHED (Int. Cl. 3) |
| | | | C 07 C 97/00<br>A 61 K 31/00 |

The present search report has been drawn up for all claims

| Place of search | Date of completion of the search | Examiner |
| --- | --- | --- |
| THE HAGUE | 27-04-1984 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82